(19) Europäisches Patentamt
European Patent Office
Office européen des brevets

(11) Veröffentlichungsnummer: **0 002 774**
**B1**

(12) **EUROPÄISCHE PATENTSCHRIFT**

(45) Veröffentlichungstag der Patentschrift: **23.09.81**

(51) Int. Cl.³: **C 07 D 501/06**, C 07 D 417/12

(21) Anmeldenummer: **78101749.6**

(22) Anmeldetag: **18.12.78**

(54) **Verfahren zur Acylierung von 7-Aminocephem-derivaten.**

(30) Priorität: **24.12.77 DE 2758000**

(43) Veröffentlichungstag der Anmeldung: **11.07.79 Patentblatt 79/14**

(45) Bekanntmachung des Hinweises auf die Patenterteilung: **23.09.81 Patentblatt 81/38**

(84) Benannte Vertragsstaaten: **BE CH DE FR GB IT NL SE**

(56) Entgegenhaltungen:
**GB-A-1 157 586**
**BODANSZKY M. & ONDETTI M. A. »Peptide Synthesis«, 1966, Interscience Publishers, New York-London-Sydney Seite 105, Zeilen 15–16**

(73) Patentinhaber: **HOECHST AKTIENGESELLSCHAFT, Postfach 80 03 20, D-6230 Frankfurt/Main 80 (DE)**

(72) Erfinder: **Blumbach, Jürgen, Dr., Rauenthaler Weg 18, D-6000 Franfurt/Main 71 (DE)**
Erfinder: **Dürckheimer, Walter, Dr., Im Lerchenfeld 45, D-6234 Hattersheim am Main (DE)**
Erfinder: **Reden, Jürgen, Dr., Bienerstrasse 10-12, D-6238 Hofheim am Taunus (DE)**
Erfinder: **Seliger, Hubert, Kallestrasse 3, D-6000 Frankfurt/Main (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 002 774 B1

BUNDESDRUCKEREI BERLIN

Verfahren zur Acylierung von 7-Aminocephemderivaten

Eine der größten Schwierigkeiten bei der Herstellung von Cephalosporinen und Penicillinen liegt in der Auffindung möglichst schonender Bedingungen für die Kupplung des Cephem- bzw. des Penam-Körpers mit einer Säurekomponente an der 7- bzw. 6-Aminogruppe. Beispielsweise führen geringe Temperatursteigerungen bei der Reaktion oder alkalische bzw. saure Reaktionsmedien zu einem raschen Abbau des $\beta$-Lactams oder bewirken tiefgreifende Veränderungen in der zu kuppelnden Säurekomponente.

Auch die vorgelagerte, notwendige Aktivierung der Säurekomponente ist oft mit nicht unerheblichen Belastungen für das Molekül verbunden und man ist oft gezwungen, aus diesem Grund erhöhte Anstrengungen zum Auffinden optimaler, schonender Reaktionsbedingungen zu unternehmen: So ist in der DOS 2 265 235 ein spezielles Verfahren angegeben, mit dem man 2-Alkoximinoaryiessigsäuren in die Säurechloride überführt, ohne daß die im Sauren sehr leicht ablaufende, unerwünschte Isomerisierung der syn- in die anti-Alkoximinoverbindung stattfindet.

Auch können die aktiven Zwischenstufen wegen ihrer Unbeständigkeit Probleme aufwerfen.

So wird in der DOS 2 540 374 ein Herstellungsverfahren für Cefazolin beschrieben, bei dem Tetrazolylessigsäure in das Säurechlorid überführt wird, welches zweckmäßigerweise wegen seiner geringen Stabilität sofort in situ umgesetzt wird. Die Kontrolle über das Maß der Aktivierung ist in einem solchen Fall erschwert.

Wie allgemein aus der Peptidchemie, insbesondere aber aus den Untersuchungen zur Darstellung beispielsweise des Ampicillins (Doyle et al. J. Chem. Soc. 1962, 1440), des Cephaloglycins (Kurita et al. J. Antibiot. 19, 243 [1966], Spencer at al., J. Med. Chem. 9, 746 [1966]) oder des Cephalexins (Ryan et al., J. Med. Chem. 12, 310 [1969]) bekannt ist, besteht bei der Verknüpfung des aktivierten und N-geschützten Phenylglycins mit dem entsprechenden $\beta$-Lactam immer das Problem einer Racemisierung am $\alpha$-C-Atom (vgl. dazu Flynn, Cephalosporins and Penicillins, Academic Press, New York, 1972, S. 86).

In den obengenannten, wie auch in vielen anderen Fällen, müssen zur Aktivierung hochreaktive Zwischenstufen wie Säureanhydride oder Säurechloride angestrebt werden. Unter Umständen ist es daher erforderlich, eventuell vorhandene, reaktive Gruppen im Säuremolekül vor der Aktivierung zu schützen, um eine unerwünschte intra- oder intermolekulare Abreaktion auszuschließen.

In der DOS 2 556 736 wird die Aktivierung einer Reihe von 2-Aminothiazolylessigsäuren und deren anschließende Kupplung mit 7-Aminocephemderivaten beschrieben, wobei die 2-Aminogruppe vor der Aktivierung durch leicht abspaltbare Schutzgruppen über teils aufwendige Verfahren blockiert und erst nach erfolgter Kupplung wieder freigesetzt wird. Dabei treten erhebliche Verluste auf.

In der französischen Patentschrift 7 601 834 wird ein Verfahren beschrieben, das 2-Tritylaminothiazolylessigsäurederivate mit 7-Aminocephalosporansäure verknüpft, und anschließend die Schutzgruppe als Tritylcarbinol sauer abspaltet.

Die Einführung solcher Schutzgruppen, wie auch deren Abspaltung nach beendeter Kupplungsreaktion ist im allgemeinen mit nicht unerheblichem Substanzverlust (s. o.), weiterer Belastung der Acylierungskomponente wie auch des Acylierungsproduktes sowie nicht unerheblichem Zeit- und Arbeitsaufwand verbunden.

In vielen Fällen ist es zudem erforderlich, das Produkt, in das die Schutzgruppe beim Abspalten überführt wird, nach dem Isolieren in eine Form zu bringen, in der es wieder als Schutzgruppenreagenz dienen und somit in den Prozeß zurückgeschleust werden kann.

Oft ist die Abspaltung der Schutzgruppe jedoch auch mit ihrem irreversiblen Verlust verbunden.

Bei den Manipulationen zur Einführung und Abspaltung eventueller Schutzgruppen leidet die Reinheit der erwünschten Substanz im allgemeinen so sehr, daß erhöhte Reinigungsanstrengungen gemacht werden müssen.

Es wurde nun gefunden, daß sich die geschilderten Schwierigkeiten umgehen lassen und Cephalosporine durch Acylierung von 7-Aminocephemderivaten in hoher Ausbeute und Reinheit dadurch gewonnen werden können, daß man die Acylierungskomponente mit einem gegebenenfalls substituierten 1-Hydroxybenzotriazol in ein aktives Derivat überführt und anschließend mit einem 7-Aminocephemderivat umsetzt.

Gegenstand der Erfindung ist somit ein Verfahren zur Acylierung von 7-Aminocephemderivaten, das dadurch gekennzeichnet ist, daß man eine Acylierungskomponente der allgemeinen Formel I

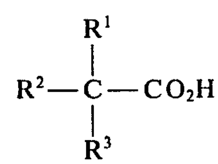

(I)

in der $R^1$ Alkyl mit 1—4 C-Atomen, Phenyl, 2-Furyl oder Thiazolyl der Formel

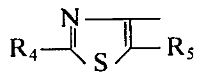

bedeutet, in der

$R_4$ für Hydroxy, Amino, Alkenylamino mit 2—4 C-Atomen, Formamino oder Acetamino, das durch Chlor, Brom, Fluor, Phenoxy oder durch Methyl oder Amino substituiertes 1,3,4-Thiadiazolylthio substituiert sein kann, steht und

$R_5$ Wasserstoff, Halogen, Cyano, Thiocyano, Hydroxy oder Alkoxycarbonyl mit 1—4 C-Atomen im Alkylteil sein kann,

$R_2$ und $R_3$ zusammen Sauerstoff oder eine Gruppe der Formel VIII

$$=N—X \quad \text{(VIII)}$$

sein können, die vorzugsweise die syn-Konfiguration hat, und in der X für Hydroxy, Alkoxy mit 1—4 C-Atomen, Benzyloxy, p-Chlorbenzyloxy, Triphenylmethoxy, Aminocarbonylmethoxy, tert.-Butoxycarbonylmethoxy, Methoxycarbonylmethoxy, Äthoxycarbonylmethoxy, Cyanomethoxy, Allyloxy, Bromallyloxy, Phenoxy oder Acyloxy mit 1—4 C-Atomen steht, mit einem 1-Hydroxybenzotriazol der allgemeinen Formel II

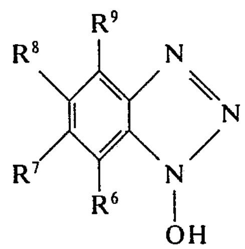

(II)

in der $R^6$, $R^7$, $R^8$ und $R^9$, die gleich oder verschieden sein können, Wasserstoff, Alkyl mit 1 bis 4 C-Atomen, Alkoxy mit 1 bis 4 C-Atomen, Halogen, Cyano, Nitro, Aminocarboxy, Alkylaminocarboxy mit 1 bis 4 Alkyl-C-Atomen, Aminosulfonyl, Alkylaminosulfonyl mit 1 bis 4 Alkyl-C-Atomen, Dialkylaminosulfonyl mit 1 bis 4 Alkyl-C-Atomen, bedeuten, in ein aktiviertes Derivat überführt und anschließend mit einem 7-Aminocephemderivat umsetzt.

Gegenstand der Erfindung ist insbesondere ein Verfahren zur Acylierung von 7-Aminocephemderivaten, das dadurch gekennzeichnet ist, daß man eine Acylierungskomponente der vorstehenden Formel I mit einem 1-Hydroxybenzotriazol der obengenannten Formel II in Gegenwart eines wasserentziehenden Mittels, insbesondere eines Carbodiimids zu einem aktivierten Derivat der Formel XIa oder XIb

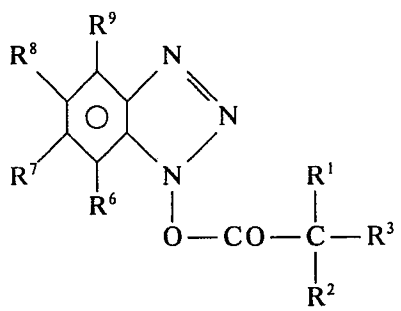

(XI a)

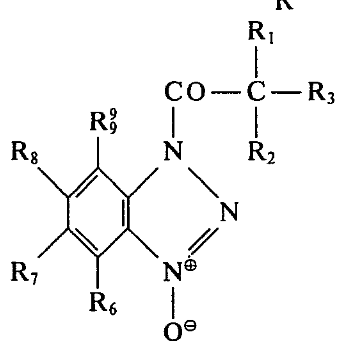

(XI b)

oder einem Gemisch davon umsetzt, dieses gegebenenfalls isoliert und es anschließend mit einem 7-Aminocephemderivat der Formel IV

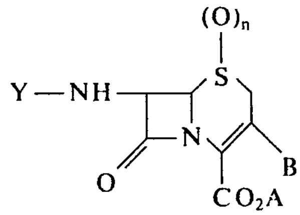

(IV)

in der n für 0, 1 oder 2, A für Wasserstoff, ein Alkali- oder Erdalkalikation, ein Kation einer Ammoniumbase oder eine leicht entfernbare Estergruppe, Y für Wasserstoff oder eine Trialkylsilylgruppe steht und B Chlor, Brom, Methoxy oder eine Gruppe der Formel IX

$$-CH_2-B' \qquad \text{(IX)}$$

bedeutet, in der B' Wasserstoff, Hydroxy, Acyloxy mit 1—4 C-Atomen, Acylthio mit 1—4 C-Atomen, Aminocarbonyloxy, 1-Pyridinium oder -S-Het bedeuten, wobei Het für einen 5-gliedrigen heterocyclischen Ring stehen kann, der 1—4 Heteroatome aus der Gruppe Stickstoff, Schwefel und Sauerstoff enthalten und durch Amino, Alkyl mit 1—4 C-Atomen oder ein durch Carboxy oder Sulfonyl substituiertes Alkyl mit 1—4 C-Atomen oder durch Thienyl substituiert sein kann oder worin Het auch einen 6-gliedrigen heterocyclischen Ring bedeuten kann, der 1 bis 3 Stickstoffatome enthalten und durch Amino, Hydroxy, Oxo, Carboxy, Carboxymethyl, Alkyl mit 1—4 C-Atomen oder Alkoxy mit 1—4 C-Atomen substituiert sein kann, wobei ein solcher heterocyclischer Ring auch noch an einen weiteren heterocyclischen Ring ankondensiert sein kann,
zu einer Verbindung der Formel VI

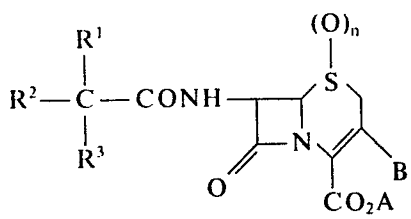

(VI)

umsetzt, in der $R^1$, $R^2$, $R^3$, A, B und n die angegebenen Bedeutungen besitzen können.

Für die erfindungsgemäße Umsetzung kommen insbesondere solche Verbindungen in Betracht, in denen die Substituenten die folgende Bedeutungen aufweisen:
$R^1$ kann stehen für Alkyl mit 1—4 C-Atomen, vorzugsweise Methyl oder Äthyl, Phenyl, 2-Furyl oder ein Thiazolyl der Formel VII

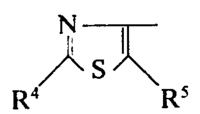

(VII)

in der $R^4$ für Hydroxy, Amino, Alkenylamino mit 2—4 C-Atomen, wie vorzugsweise Allylamino, Formamido oder Acetamido, das durch Chlor, Brom, Fluor, Phenoxy oder durch Methyl oder Amino substituiertes 1,3,4-Thiadiazolylthio substituiert sein kann, wie insbesondere Chloracetamido, Bromacetamido, Trifluoracetamido, Phenoxyacetamido, (5-Amino-1,3,4-thiadiazol-2-yl)-thioacetamido oder (5-Methyl 1,3,4-thiadiazol-2-yl)-thioacetamido, und
$R^5$ für Wasserstoff, Halogen, wie vorzugsweise Fluor, Chlor, Brom, Cyano, Thiocyano, Hydroxy oder Alkoxycarbonyl mit 1—4 C-Atomen im Alkylrest, wie vorzugsweise Methoxycarbonyl oder Äthoxycarbonyl steht.

$R^2$ und $R^3$ können zusammen Sauerstoff oder vorzugsweise eine Gruppe der Formel VIII

$$=N-X \qquad \text{(VIII)}$$

bedeuten, in der X vorzugsweise die syn-Konfiguration hat und für Hydroxy, Alkoxy mit 1—4 C-Atomen, wie vorzugsweise Methoxy- Äthoxy, Propoxy, Butoxy, Benzyloxy, p-Chlorbenzyloxy, Triphenylmethoxy, Aminocarbonylmethoxy, tert.-Butoxycarbonylmethoxy, Methoxycarbonylmethoxy, Äthoxycarbonylmethoxy, Cyanomethoxy, Allyloxy, Bromallyloxy, Phenoxy oder Acyloxy mit 1—4 C-Atomen, wie vorzugsweise Acetoxy, Chloracetoxy oder Bromacetoxy steht.

$R^6$, $R^7$, $R^8$ und $R^9$ können gleich oder verschieden sein und die Bedeutung besitzen von Wasserstoff, Alkyl mit 1—4 C-Atomen, vorzugsweise Methyl oder Äthyl, Alkoxy mit 1—4 C-Atomen, vorzugsweise Methoxy oder Äthoxy, Halogen, wie z. B. Brom, Chlor oder Fluor, Cyano, Nitro, Aminocarboxy, Alkylaminocarboxy mit 1—4 Alkyl-C-Atomen, wie vorzugsweise Methylaminocarboxy, Äthylaminocarboxy, Aminosulfonyl, Alkylaminosulfonyl mit 1—4 C-Atomen, vorzugsweise Methylaminosulfonyl, Dialkylaminosulfonyl mit 1—4 C-Atomen pro Alkylgruppe, vorzugsweise Dimethylaminosulfonyl oder Diäthylaminosulfonyl.

Besonders bevorzugte Verbindungen der Formel VI sind: 1-Hydroxybenzotriazol, 1-Hydroxy-4-methyl-benzotriazol, 1-Hydroxy-5-methyl-benzotriazol, 1-Hydroxy-6-methyl-benzotriazol, 1-Hydroxy-5,6-dimethyl-benzotriazol, 1-Hydroxy-5-methoxy-benzotriazol, 1-Hydroxy-6-methoxy-benzotriazol, 5-Chlor-1-hydroxy-benzotriazol, 5,6-Dichlor-1-hydroxy-benzotriazol, 6-Brom-1-hydroxy-benzotriazol, 4,5,6,7-Tetrachlor-1-hydroxy-benzotriazol, 6-Chlor-1-hydroxy-5-methyl-benzotriazol, 6-Chlor-1-hydroxy-5-isopropyl-benzotriazol,4-Chlor-6-nitro-1-hydroxy-7-methyl-benzotriazol, 6-Nitro-4-methyl-1-hydroxy-benzotriazol, 6-Nitro-1-hydroxy-benzotriazol, 1-Hydroxy-6-methyl-5-cyano-benzotriazol, 1-Hydroxybenzotriazol-carbonsäure-(6)-amid, 1-Hydroxy-6-methylaminosulfonyl-benzotriazol, 1-Hydroxy-6-aminosulfonyl-benzotriazol oder 1-Hydroxy-6-diäthylaminosulfonyl-benzotriazol, insbesondere das 1-Hydroxy-benzotriazol.

A kann stehen für Wasserstoff, ein Alkali- oder Erdalkalikation, insbesondere Natrium, Kalium oder Calcium, ein Kation einer Ammoniumbase, wie z. B. ein Trialkylammonium, vorzugsweise Trimethylammonium, Triäthylammonium, Tri-n-butyl-ammonium oder eine leicht entfernbare Estergruppe, wie insbesondere Methyl, Benzyl, Benzhydryl, Pivaloyloxymethyl, 2,2,2-Trichloräthyl, tert.-Butyl oder Trimethylsilyl.

B kann stehen für Chlor, Brom, Methoxy oder für eine Gruppe der Formel IX

$$-CH_2-B' \qquad (IX)$$

in der B' die Bedeutung haben kann von Wasserstoff, Hydroxy, Acyloxy mit 1—4 C-Atomen, wie vorzugsweise Acetoxy, Acylthio mit 1—4 C-Atomen, wie vorzugsweise Acetylthio, Aminocarbonyloxy, 1-Pyridinium oder von -S-Het, worin Het ein 5-gliedriger heterocyclischer, 1—4 Heteroatome aus der Gruppe Stickstoff, Schwefel und Sauerstoff enthaltender Ring sein kann, insbesondere Pyrolyl, Pyrazolyl, Imidazolyl, Triazolyl, Tetrazolyl, Thiazolyl, Isothiazolyl, Thiadiazolyl, Oxazolyl, Isoxazolyl oder Oxadiazolyl, der noch substituiert sein kann durch Thienyl, Amino oder Alkyl mit 1—4 C-Atomen, vorzugsweise Methyl, wobei der Alkylsubstituent noch weiter substituiert sein kann durch Carboxy oder Sulfonyl. Als besonders bevorzugte Reste seien hierfür beispielsweise erwähnt:

1-Methyltetrazolyl, 1-Carboxymethyl-tetrazolyl, 1-Sulfonylmethyl-tetrazolyl, 5-Amino-1,3,4-triazolyl, 5-Methyl-1,3,4-triazolyl, 5-(2-Thienyl)-1,3,4-triazolyl, 5-(2-Thienyl)-1-methyl-1,3,4-triazolyl, 1-Carboxymethyl-1,3,4-triazolyl, 1-Carboxymethyl-5-methyl-1,3,4-triazolyl, 5-Methyl-1,3,4-thiadiazolyl, 5-Amino-1,3,4-thiadiazolyl, 4-Methylthiazolyl, 4-Carboxymethylthiazolyl, 5-Aminothiazolyl, 3-Methyl-1,2,4-thiadiazolyl, 4,5-Dimethylthiazolyl.

In dem vorstehend aufgeführten Rest $-CH_2$-S-Het kann Het auch ein 6-gliedriger heterocyclischer Ring sein, der 1—3 Stickstoffatome enthält. Insbesondere seien als Beispiele genannt Pyridyl, Pyrimidinyl, Pyrazinyl und Pyridazinyl, wobei diese 6-gliedrigen heterocyclischen Reste auch noch substituiert sein können durch Alkyl mit 1—4 C-Atomen, vorzugsweise Methyl, Alkoxy mit 1—4 C-Atomen, vorzugsweise Methoxy oder Äthoxy, Amino, Hydroxy, Oxo, Carboxy oder Carboxymethyl. Als besonders bevorzugte Reste seien beispielsweise erwähnt 4,6-Diamino-pyrimidin-2-yl, 6-Hydroxy-4-aminopyrimidin-2-yl, 4,5-Diamino-6-hydroxy-pyrimidin-2-yl, 5-Carboxy-methyl-4-hydroxy-6-methylpyrimidin-2-yl.

Als heterocyclischer Ring, der an den Heterocyclus ankondensiert sein kann, kommen insbesondere ein Furo-, Thieno- oder Pyridoring in Betracht. Als bevorzugtes Beispiel hierfür sei erwähnt 5,6-Benzo-1,1-dioxo-1,2,4-thiadiazin-3-yl.

Y kann für Wasserstoff stehen oder eine Trialkylsilylgruppe, insbesondere für Trimethylsilyl.

Als Carbodiimid, das als wasserentziehendes Mittel für die Umsetzung der Acylierungskomponente mit dem Hydroxybenzotriazol eingesetzt wird, können die für derartige Umsetzungen geeigneten Carbodiimide verwendet werden, insbesondere solche der allgemeinen Formel X

$$R^{10}-N=C=N-R^{11} \qquad (X)$$

in der $R^{10}$ und $R^{11}$ die folgende Bedeutung haben können. $R^{10}$ und $R^{11}$ können gleich oder verschieden sein und stehen für Alkyl mit 1—4 C-Atomen, wie vorzugsweise Methyl, Äthyl, Propyl, Isopropyl oder Butyl, für einen gegebenenfalls substituierten Aminoalkyl mit 1—4 C-Atomen pro Alkylgruppe, wie vorzugsweise 3-Dimethylamino-propyl, 3-Triäthylaminopropyl, 2-N-Morpholinoäthyl, 2-Pyrrolidinoäthyl, sowie deren Methojodide und Metho-p-toluol-sulfonate, für einen gegebenenfalls substituierten Cycloalkylrest, wie vorzugsweise Cyclohexyl oder für einen gegebenenfalls substituierten aromatischen Rest, wie vorzugsweise Phenyl, p-Chlorphenyl, p-Tolyl, m-Chlor-phenyl oder m-Tolyl.

Die Ausgangsmaterialien für die erfindungsgemäße Umsetzung sind literaturbekannt oder lassen sich nach literaturbekannten Verfahren herstellen.

Die Umsetzung der Carbonsäure der Formel I mit einem gegebenenfalls substituierten 1-Hydroxy-benzotriazol der Formel II kann unter variierbaren experimentellen Bedingungen erfolgen. So ist es beispielsweise möglich, ein Gemisch aus der Carbonsäure I, gelöst oder suspendiert in einem geeigneten Lösungsmittel, und einen gegebenenfalls substituierten 1-Hydroxybenzotriazol II innerhalb eines größeren Temperaturbereiches mit einem Carbodiimid, gelöst in einem geeigneten Lösungsmittel oder in Substanz, zu vereinigen.

Das Benzotriazol der Formel II kann in etwa äquivalenten Mengen, vorzugsweise jedoch im Überschuß von beispielsweise etwa 0,2 bis 1 Mol oder mehr eingesetzt werden.

Der Fortgang der Umsetzung kann in an sich bekannter Weise verfolgt werden, so beispielsweise durch Dünnschicht- oder Hochdruck-Flüssigkeitschromatographie, durch Kernresonanz- oder Infrarot-Spektroskopie.

Die aktivierten Carbonsäuren der Formel XI können, falls erwünscht, mit an sich allgemein bekannten Methoden wie Extraktion, Ausfällung oder fraktionierte Kristallisation isoliert werden. In vielen Fällen kann man auch die bei ihrer Herstellung anfallenden Reaktionslösungen, gegebenenfalls nach Entfernen des als Reaktionsprodukt entstandenen Harnstoffs, direkt im Sinne des erfindungsgemäßen Verfahrens weiter umsetzen.

Die aktivierten Carbonsäuren können in zwei Formen vorliegen, dem »aktivierten Ester« der allgemeinen Formel XIa und dem »aktivierten Amid« der allgemeinen Formel XI b, die in Lösung offensichtlich im Gleichgewicht stehen.

Im festen Zustand liegt im allgemeinen der »aktivierte Ester« der allgemeinen Formel XIa vor. Je nach Substanz können aber auch der »aktivierte Ester« und das »aktivierte Amid« getrennt isoliert werden.

Die Isolierung des »aktivierten Esters« und des »aktivierten Amids« aus dem Reaktionsmedium und ihre Trennung kann nach an sich bekannten Methoden erfolgen, die z. B. auf ihrer unterschiedlichen Löslichkeit, auf ihrer unterschiedlichen Polarität oder Kristallinität beruhen:

So läßt sich beispielsweise der aktivierte Ester der $\alpha$-syn-Methoximino-$\alpha$-(2-amino-thiazol-4-yl)-essigsäure von dem entsprechenden Amid durch fraktioniertes Ausfällen aus Dimethylformamidlösung durch Zugabe von Wasser abtrennen. Auch eine chromatographische Trennung, wie beispielsweise an Silicagel kann sich als angezeigt erweisen.

Wird ein Amin, wie z. B. Verbindungen der Formel IV, mit dem »aktivierten Ester« der Formel XI a bzw. mit dem »aktivierten Amid« der Formel XI b umgesetzt, so erhält man in beiden Fällen identische Produkte.

In der Regel ist aber weder die Isolation der aktivierten Carbonsäure noch eine Trennung in den »aktivierten Ester« und das »aktivierte Amid« für das erfindungsgemäße Verfahren notwendig.

Im vorliegenden Text wird daher unter einer »aktivierten Carbonsäure« das Reaktionsprodukt im Sinne des erfindungsgemäßen Verfahrens zwischen einer Carbonsäure der Formel I und einem gegebenenfalls substituierten 1-Hydroxy-benzotriazol der Formel II verstanden, unabhängig davon, ob es sich um eine der beiden reinen Formen — »aktivierter Ester« XIa oder »aktiviertes Amid« XI b — oder um eine Mischung der beiden Formen handelt.

Als Lösungsmittel eignen sich die in der Penicillin- und Cephalosporinchemie gebräuchlichen Lösungsmittel oder Lösungsmittelgemische, wie z. B. Dimethylformamid, Dimethylacetamid, Diäthyläther, Di-iso-propyläther, Tetrahydrofuran, Dioxan, Tetrachlorkohlenstoff, Chloroform, Methylenchlorid, Acetonitril, Essigester, Aceton oder Butanon.

Bevorzugte Lösungsmittel sind Dimethylformamid, Tetrahydrofuran, Methylenchlorid, Acetonitril und Essigester, sowie Gemische derselben.

Zu achten ist lediglich darauf, daß das Lösungsmittel mit den Reaktanten nicht in Reaktion tritt, wie es z. B. bei hydroxylgruppenhaltigen Lösungsmitteln, wie beispielsweise $H_2O$ der Fall ist.

Es sind aber auch schon Aktivierungen erfolgreich durchgeführt worden, bei denen Säuren eingesetzt wurden, die niedrige Alkanole, wie Methanol oder Äthanol, in Form von Addukten gebunden enthielten und/oder eine alkoholische Hydroxygruppe im Molekül enthielten.

Es ist hierbei lediglich darauf zu achten, daß keine Basen anwesend sind, die zu einer im Sinne des erfindungsgemäßen Verfahrens unerwünschten Reaktion zwischen der aktivierten Säure und dem Alkohol führen (vgl. dazu Itoh et al., Synthesis 1975, 456).

Die Reaktionstemperatur für die Umsetzung der Carbonsäure mit dem gegebenenfalls substituierten 1-Hydroxy-benzotriazol liegt vorteilhaft zwischen etwa $-20$ und etwa $+60°$ C, bevorzugt bei $-10$ bis $+40°$ C, insbesondere zwischen 0 bis 25° C.

Die Reaktionsdauer liegt im allgemeinen zwischen etwa 10 min und etwa 24 Stunden, vorzugsweise im Bereich von 1 bis 4 Stunden.

Als Carbodiimide, die bevorzugt als wasserentziehende Mittel eingesetzt werden, können besonders gut die aus der Paptidchemie und die aus der Penicillin- bzw. Cephalosporinchemie bekannten Verbindungen verwendet werden, insbesondere das N,N'-Dicyclohexylcarbodiimid und N,N'-Diisopropylcarbodiimid.

Der bei der Aktivierung aus dem Carbodiimid entstehende Harnstoff kann abgetrennt werden,

besonders wenn beabsichtigt ist, die aktivierte Carbonsäure anschließend zu isolieren.

Man kann aber auch ohne Nachteil auf die Abtrennung verzichten und die nachfolgende Umsetzung mit dem 7-Aminocephemderivat in seiner Gegenwart durchführen.

Die Umsetzung der aktivierten Carbonsäure mit 7-Aminocephemderivaten kann unter variierbaren experimentellen Bedingungen durchgeführt werden. So ist es beispielsweise möglich, die aktivierte Carbonsäure sowohl in Substanz, als auch nach ihrer Isolation in einem geeigneten Lösungsmittel gelöst oder suspendiert zu einer Lösung des 7-Aminocephemderivates zu geben. Es ist jedoch auch möglich, sowohl die von dem aus dem Carbodiimid entstandenen Harnstoff befreite, als auch die diesen Harnstoff noch enthaltende Lösung der aktivierten Carbonsäure mit einer Lösung des 7-Aminocephemderivates zu vereinigen.

Als Lösungsmittel für die Acylierungsreaktion eignen sich vor allem die in der Penicillin- bzw. Cephalosporinchemie gebräuchlichen Lösungsmittel oder Lösungsmittelgemische.

So kann die Reaktion beispielsweise in Amiden, wie vorzugsweise Dimethylformamid oder Dimethylacetamid, in Äthern, wie vorzugsweise Di-isopropyläther, Diäthyläther, Tetrahydrofuran und Dioxan, chlorierten Kohlenwasserstoffen, wie vorzugsweise Tetrachlorkohlenstoff, Chloroform und $CH_2Cl_2$, in Nitrilen, wie vorzugsweise Acetonitril, in Estern, wie vorzugsweise Essigsäureäthylester oder in Ketonen, wie vorzugsweise Aceton und Butanon durchgeführt werden.

Es können aber auch Gemische dieser Lösungsmittel zum Einsatz kommen.

Besonders bevorzugte Lösungsmittel sind Dimethylformamid, Tetrahydrofuran, Methylenchlorid, Acetonitril und Essigester sowie Gemische derselben.

Überraschend war die Feststellung, daß keine besonderen Maßnahmen getroffen werden müssen, um die Reaktion unter völlig wasserfreien Bedingungen durchzuführen. Der geringe Wassergehalt der handelsüblichen Lösungsmittel wirkt sich nicht nachteilig auf die Reaktion aus. Auch die Anwesenheit von Verunreinigungen anderer hydroxylgruppenhaltiger Lösungsmittel, wie niedermolekularer Alkanole, beispielsweise Methanol oder Äthanol beeinträchtigen die Reaktion nicht.

Zur guten Reaktionsführung ist es zweckmäßig, die 7-Aminocephemderivate in Lösung einzusetzen. Als für die Auflösung in den obengenannten Lösungsmitteln bzw. Lösungsmittelgemischen geeignet hat sich ein Zusatz von organischen Basen, insbesondere von tertiären Aminen, wie z. B. Trimethylamin, Triäthylamin, Tri-n-butylamin, N,N-Dimethylanilin oder N-Methylmorpholin erwiesen. Die Basen werden im allgemeinen in mindestens stöchiometrischen Mengen zugesetzt. Ein Überschuß an Base von beispielsweise etwa 0,1 bis etwa 2,0, insbesondere 0,2 bis 0,8 Mol, kann für die Umsetzung von Vorteil sein.

Auch Ester der 7-Aminocephemsäure können in der Reaktion eingesetzt werden. Vorzugsweise werden dabei leicht abspaltbare Ester, wie z. B. der Benzyl-, Methyl-, der Pivaloyloxymethyl-, der Benzhydryl-, der 2,2,2-Trichloräthyl-, der tert.-Butyl- oder ein Trialkylsilylester, insbesondere der Trimethylsilylester verwendet. Der Vorteil vor allem der letztgenannten Trialkylsilylester, wie beispielsweise des Trimethylsilylesters, besteht in seiner leichten Abspaltung, die oft schon beim Aufarbeiten des Reaktionsproduktes erfolgt.

Die aktivierten Carbonsäuren werden zur Erzielung hoher Ausbeuten in mindestens stöchiometrischen Mengen eingesetzt. Ein Überschuß von etwa 5 bis 25% kann sich als zweckmäßig erweisen.

Die Dauer der Umsetzung zwischen der aktivierten Carbonsäure und dem 7-Aminocephemderivat liegt im allgemeinen zwischen etwa $^1/_2$ Stunde und 25 Stunden, vorzugsweise zwischen 2 und 16 Stunden.

Die Reaktion kann in einem weiten Temperaturbereich durchgeführt werden, beispielsweise zwischen etwa $-20$ und $+60°$ C, wobei jedoch zweckmäßigerweise eine Temperatur von 40° C nicht überschritten werden sollte. Vorzugsweise liegt die Reaktionstemperatur zwischen 0 und $+25°$ C.

Die Isolierung der Verbindungen der Formel VI aus dem Reaktionsmedium kann nach an sich bekannten Methoden, die sich nach der Löslichkeit der erhaltenen Verbindungen richten, erfolgen.

So lassen sich beispielsweise die Reaktionsprodukte gegebenenfalls nach Abdampfen des organischen Lösungsmittels in Wasser aufnehmen und nach entsprechenden Reinigungsoperationen, wie z. B. Filtrieren oder Zentrifugieren, durch Zugabe von Säuren ausfällen. Zum Entfernen des bei der Reaktion gebildeten Hydroxybenzotriazols wird das Produkt zweckmäßigerweise in einem geeigneten Lösungsmittel, wie beispielsweise Methanol, Äthanol, Propanol, Butanol oder Aceton aufgerührt.

Gegebenenfalls kann das Hydroxybenzotriazol auch bei einem pH zwischen etwa 5,0 und 3,5 aus der wäßrigen Lösung des Reaktionsproduktes in ein mit Wasser nicht mischbares organisches Lösungsmittel, beispielsweise Essigester oder Methylisobutylketon, extrahiert werden. Durch Abdampfen des Lösungsmittels gewinnt man das in die Reaktion eingesetzte Hydroxybenzotriazol zurück.

Durch anschließendes weiteres Ansäuern fällt man die Produkte der Formel VI in Form ihrer freien Säuren aus.

Sie können durch Filtration gewonnen werden, oder gegebenenfalls in mit Wasser nicht mischbare organische Lösungsmittel, wie beispielsweise Essigester oder Methylisobutylketon, extrahiert werden.

Aus den Extrakten können die entstandenen Produkte der Formel VI beispielsweise durch Abdampfen des Lösungsmittels und Anreiben mit Äther gewonnen werden.

Auch eine präparative chromatographische Abtrennung kann angezeigt sein.

Bei der erfindungsgemäßen Umsetzung anfallende Ester, deren Estergruppe eine Schutzfunktion für die Carboxylgruppe hatte, wie beispielsweise p-Methoxybenzyl, p-Nitrobenzyl oder tert.-Butyl, können nach üblichen experimentellen Methoden, die von ihrer Löslichkeit, ihrer Kristallinität oder ihrer Extrahierbarkeit abhängen, vom gegebenenfalls substituierten 1-Hydroxybenzotriazol abgetrennt werden. Falls erwünscht können sie dann in literaturbekannter Weise in die freien Carbonsäuren oder in deren Salze überführt werden.

Das erfindungsgemäße Verfahren zeichnet sich somit dadurch aus, daß es unter sehr milden Reaktionsbedingungen zu äußerst reaktionsfähigen Carbonsäurederivaten führt, und es damit möglich wird, sowohl sehr empfindliche, als auch aufgrund sterischer oder elektronischer Effekte schwer aktivierbare Carbonsäuren in hochreaktive Acylierungsreagentien umzuwandeln.

So gelingt überraschenderweise mit einigen nach dem erfindungsgemäßen Verfahren hergestellten aktiven Carbonsäurederivaten, beispielsweise mit dem Reaktionsprodukt zwischen 1-Hydroxybenzotriazol und $\alpha$-(2-Aminothiazol-4-yl)$\alpha$-synmethoximinoessigsäure, die Übertragung der Acylkomponente auf 7-Aminocephemderivate, die bei der Verwendung der mit dem in der Cephalosporinchemie gebräuchlichen N-Hydroxysuccinimid (vgl. z. B. GB-A 1 157 586) als N-Acyloxysuccinimid aktivierten Carbonsäure, beispielsweise mit N-[$\alpha$-(2-Amino-thiazol-4-yl)-$\alpha$-syn-methoximino-acetoxy]-succinimid auch bei Anwendung drastischer Reaktionsbedingungen, wie verlängerter Reaktionszeit und erhöhter Temperatur, versagt.

Überraschend war weiterhin die Feststellung, daß es bei der Aktivierung im Sinne des erfindungsgemäßen Verfahrens bei solchen aminosubstituierten Carbonsäuren keiner Aminoschutzgruppe bedarf, bei der die Aminogruppe einen $pK_s$-Wert von kleiner als 6,0 besitzt: Die oben erwähnten negativen Aspekte der Verwendung einer Schutzgruppe, wie zusätzliche Syntheseschritte, zusätzliche Belastung der Molekel, Ausbeuteverminderung, höherer Zeitaufwand, höherer Reinigungsaufwand und Reaktivierung des Schutzgruppenreagenzes entfallen bei dem erfindungsgemäßen Verfahren daher vollständig.

Auch Hydroxylgruppen werden überraschenderweise von den mit gegebenenfalls substituierten 1-Hydroxybenzotriazolen aktivierten Carbonsäuren nicht angegriffen, obwohl letztere nach McCarthy et al. J. Chem. Soc. Perkin II 1977, 224 in ihrer Reaktivität mit Anhydriden verglichen werden können.

Dies gilt auch für alkoholische Hydroxylgruppen:

Es ist daher überraschenderweise auch möglich, solche Carbonsäuren mit Erfolg im Sinne des erfindungsgemäßen Verfahrens zu aktivieren, die Alkanole adduktartig im Kristall gebunden enthalten oder anderweitig mit diesen verunreinigt sind.

In der französischen Patentschrift 76 01 834 wird ein Verfahren beschrieben, bei dem $\alpha$-[2-Tritylamino-thiazol-4-yl]-$\alpha$-syn-methoximino-essigsäure in ihr symmetrisches Anhydrid überführt und unter Ausnutzung theoretisch nur maximal der Hälfte der eingesetzten Carbonsäure mit 7-Amino-cephalosporansäure gekuppelt wird.

Das erfindungsgemäße Verfahren zeichnet sich im Vergleich dazu zum einen dadurch aus, daß, wie oben ausgeführt, keine Aminoschutzgruppe für die Säure benötigt wird und zum anderen der zwangsläufige Verlust der Hälfte der Carbonsäure umgangen wird.

Die bei der Umsetzung von gegebenenfalls substituierten 1-Hydroxybenzotriazolen der Formel II mit einer Carbonsäure der Formel I erhaltenen aktivierten Derivate XI a und/oder XI b sind für die Synthese von Cephalosporinen daher sehr wertvoll, weil sie — wie oben im einzelnen ausgeführt — u. a. als hochreaktive Verbindungen unter sehr milden Bedingungen gebildet werden, weil ihre Herstellung, Handhabung und Umsetzung äußerst problemlos ist, weil sie unter sehr milden Bedingungen mit Cephalosporinen in der gewünschten Weise reagieren, und weil sie bei schwach basischen Aminogruppen und bei Hydroxylgruppen eine Schutzgruppe überflüssig machen.

Die folgenden Beispiele erläutern die Erfindung, ohne sie jedoch darauf einzuschränken.

## Beispiel 1

### 7-$\beta$-[$\alpha$-syn-Methoximino-$\alpha$-(2-amino-thiazol-4-yl)-acetamido]-cephalosporansäure

### Variante A

#### 1. Stufe

60,3 g $\alpha$-(2-Amino-thiazol-4-yl)-$\alpha$-syn-methoximinoessigsäure werden in 540 ml DMF gelöst und mit 40,5 g Hydroxybenzotriazol, gelöst in 240 ml DMF, versetzt. Unter Rühren werden 61,89 g Dicyclohexylcarbodiimid zugegeben. Nach 2 Stunden wird vom ausgefallenen Dicyclohexylharnstoff abfiltriert und der Filterrückstand mit 20 ml DMF gewaschen. Das Filtrat wird auf 10°C gekühlt und unter Rühren tropfenweise mit 500 ml $H_2O$ versetzt. Der sich bildende Niederschlag wird abgesaugt, mit $H_2O$ gewaschen und über $P_2O_5$ im Vakuum getrocknet:

Man erhält so 77,1 g eines Gemisches aus

1-[α-syn-Methoximino-α-(2-amino-thiazol-4-yl)-acetoxy]-benzotriazol
und
1-[α-syn-Methoximino-α-(2-amino-thiazol-4-yl)-acetyl]-benzotriazol-3-oxid
mit dem Schmelzpunkt 145—147° C.

2. Stufe

68,08 g 7-Aminocephalosporansäure werden in 600 ml Methylenchlorid aufgerührt und mit 75,75 g Triäthylamin versetzt. Es wird bis zur klaren Lösung gerührt und 79,58 g des in Stufe 1 gewonnenen Gemisches, suspendiert in 600 ml Tetrahydrofuran, portionsweise zugegeben. Die Lösung wird über Nacht gerührt, wonach der Aktivester umgesetzt ist.

Die Reaktionslösung wird eingedampft und der Rückstand in einem Gemisch aus 350 ml Ameisensäure (100%) und 100 ml $H_2O$ aufgenommen. Diese Lösung wird langsam in eine Lösung aus 875 g Ammoniumsulfat in 1750 ml $H_2O$ unter Rühren eingetropft.

Nach 1 Stunde Rühren wird der helle, körnige Niederschlag abfiltriert, mit $H_2O$ gewaschen, getrocknet, und in 500 ml Äthanol 1/2 Stunde bei 50° C und 1 Stunde bei R. T. gerührt. Es wird abfiltriert und noch zweimal mit je 50 ml Äthanol gewaschen. Nach Trocknen über $P_2O_5$ ergeben sich 78,5 g der Titelverbindung.

IR (KBr): 1770 $cm^{-1}$ (β-Lactam)
NMR ($DMSO-d_6$) : δ=2,05 ppm (s, 3H, $OCOCH_3$)
δ=3,85 ppm (s, 3H, $OCH_3$)
δ=6,75 ppm (s, 1H, Thiazol-H).

Variante B

1,0 g α-(2-Amino-thiazol-4-yl)-α-syn-methoximino-essigsäure werden in 25 ml Tetrahydrofuran aufgeschlämmt, mit 0,676 g Hydrobenzotriazol und anschließend mit 1,03 g Dicyclohexylcarbodiimid versetzt. Man rührt 2 Stunden, saugt vom entstandenen Dicyclohexylharnstoff ab und versetzt diese Lösung tropfenweise bei Raumtemperatur mit einer Lösung aus 1,36 g 7-Aminocephalosporansäure und 1,5 g Triäthylamin in 25 ml Methylenchlorid. Man rührt 16 Stunden bei Raumtemperatur, filtriert vom Ungelösten ab und dampft ein. Der Rückstand wird in 25 ml Wasser aufgenommen, und bei pH 4,5 dreimal mit je 10 ml Essigester ausgeschüttelt. Die wäßrige Phase wird mit 2 n-Salzsäure bei 0° C auf pH 2,5 angesäuert. Man filtriert, trocknet über $P_2O_5$ im Vakuum und erhält 1,07 g 7-[α-syn-Methoximino-α(2-aminothiazol-4-yl)-acetamido]-cephalosporansäure. Dieses Produkt ist im NMR-Spektrum (aufgenommen in $DMSO-d_6$) mit der gemäß Beispiel 1, Variante A hergestellten Verbindung identisch.

Variante C

Stufe 1

1-[α-syn-Methoximino-α-(2-amino-thiazol-4-yl)-acetoxy]-benzotriazol

und

3-[α-syn-Methoximino-α-(2-amino-thiazol-4-yl)-acetyl]-benzotriazol-1-oxid

8,6 g α-syn-Methoximino-α-(2-amino-thiazol-4-yl)-essigsäure, gelöst in 80 ml Dimethylformamid wurden bei 0° mit 5,8 g 1-Hydroxybenzotriazol, gelöst in 40 ml Dimethylformamid, und anschließend mit 8,9 g festem N,N-Dicyclohexylcarbodiimid versetzt. Nach 5-stündigem Rühren im Eisbad wurde der gebildete Dicyclohexylharnstoff abgesaugt.

Zu der Mutterlauge wurden bei 0° 60 ml $H_2O$ zugetropft. Nach 10 min wurden 7,14 g 1-[α-syn-Methoximino-α-(2-amino-thiazol-4-yl)-acetoxy]-benzotriazol isoliert.

F: 153—155 (Z)
$R_F$: 0,58 (Chloroform/Aceton=2/3)
IR (KBr): 1805 $cm^{-1}$ (Ester)
NMR ($DMSO-d_6$): δ=4,07 ppm (s, 3H, $OCH_3$)
δ=7,23 ppm (s, 1H, Thiazol-H)
δ=7,0—8,5 ppm (m, 7H, $NH_2$, Benzo-H, Thiazol-H)

Aus der Mutterlauge kristallisierten nach 40 min bei 0° C 3,98 g 3-[α-syn-Methoximino-α-(2-amino-thiazol-4-yl)-acetyl]-benztriazol-1-oxid × $1^1/_2$ $H_2O$ aus

F: 151—153 (Z)
Misch-Fp beider Produkte: 148—149 (Z)
$R_f$: 0,52 (Chloroform/Aceton = 2/3)
IR (KBr): 1725 $cm^{-1}$ (Amid)
NMR (DMSO-$d_6$): $\delta$ = 3,87 ppm (s, 3H, $OCH_3$)
$\delta$ = 7,15 ppm (s, 1H, Thiazol-H)
$\delta$ = 7,22 ppm (s, 2H, $NH_2$)
$\delta$ = 7,5—8,55 ppm (m, 4H, Benzo-H)
$\delta$ = 3,25 ppm (s, 3H, $H_2O$)

## Stufe 2a

Zu einer bei Raumtemperatur gerührten Lösung von 1,365 g 7-Amino-cephalosporansäure und 1,515 g Triäthylamin in 20 ml Methylenchlorid werden 1,593 g 1-[α-syn-Methoximino-α-(2-amino-thiazol-4-yl)-acetoxy]-benzotriazol, suspendiert in 20 ml Tetrahydrofuran gegeben. Nach kurzer Zeit entsteht eine klare Lösung, die bei Raumtemperatur über Nacht gerührt wird. Die Reaktionslösung wird nach Filtration eingedampft und der Rückstand in einem Gemisch aus 7 ml Ameisensäure (100%) und 2 ml $H_2O$ aufgenommen. Die Lösung wird langsam zu einer Lösung von 17,5 g $(NH_4)_2SO_4$ in 35 ml $H_2O$ getropft. Es wird vom Niederschlag abfiltriert und in 30 ml Äthanol 1/2 Stunde bei 55° und 1 Stunde bei Raumtemperatur gerührt. Es wird abfiltriert und in 30 ml Äthanol 1/2 Stunde bei 55° und 1 Stunde bei Raumtemperatur gerührt. Es wird abfiltriert und über $P_2O_5$ im Vakuum getrocknet. Man erhält so 7-β-[α-syn-Methoximino-α-(2-amino-thiazol-4-yl)-acetamido]-cephalosporansäure, dünnschichtchromatographisch IR- und NMR-spektroskopisch identisch mit dem in Variante A erhaltenen Produkt.

## Stufe 2b

Verwendet man in Variante C, Stufe 2a 1,593 g 3-[α-syn-Methoximino-α-(2-amino-thiazol-4-yl)-acetyl]-benzotriazol-1-oxid, so erhält man ebenfalls 7-β-[α-syn-Methoximino-α-(2-amino-thiazol-4-yl)-acetamido]-cephalosporansäure, dünnschichtchromatographisch, IR- und NMR-spektroskopisch identisch mit dem in Variante C, Stufe 2a gewonnenen Produkt.

## Beispiel 2

### 7-β-[α-syn-Äthoximino-α-(2-amino-thiazol-4-yl)-acetamido]-cephalosporansäure

Gemäß Beispiel 1 B bei Verwendung von 1,07 g α-(2-Aminothiazol-4-yl)-α-syn-äthoximino-essigsäure. Man isoliert 0,98 g der Titelverbindung.

F: 125—135 (Z)
IR (KBr): 1770 $cm^{-1}$ (β-Lactam)
1720 $cm^{-1}$ (Acetat)
NMR (DMSO-$d_6$) $\delta$ = 2,0 ppm (s, 3H, $OCO\underline{CH_3}$)
$\delta$ = 1,27 ppm (t, 3H, $C\underline{H_3}$)
$\delta$ = 4,13 ppm (q, 2H, $OCH_2$—)
$\delta$ = 6,8 ppm (s, 1H, Thiazol-H)

## Beispiel 3

### 7-β-[α-syn-Propoximino-α-(2-amino-thiazol-4-yl)-acetamido]-cephalosporansäure

Gemäß Beispiel 1 B bei Verwendung von 1,15 g α-(2-Aminothiazol-4-yl)-α-syn-propoximino-essigsäure. Man isoliert 0,8 g der Titelverbindung.

F = 120—130 (Z)
IR (KBr): 1775 $cm^{-1}$ (β-Lactam)
1720 $cm^{-1}$ (Acetat)
NMR (DMSO-$d_6$): $\delta$ = 1,23 ppm (t, 3H, $CH_3$)
$\delta$ = 2,05 ppm (s, 3H, $CO\underline{CH_3}$)

δ=4,00 ppm (t, 2H, $O-CH_2$)
δ=6,70 ppm (s, 1H, Thiazol-H)

Beispiel 4

7-β-[α-syn-Isopropoximino-α-(2-amino-thiazol-4-yl)-acetamido]-cephalosporansäure

Gemäß Beispiel 1B bei Verwendung von 1,15 g α-(2-Amino-thiazol-4-yl)-α-syn-isopropoximino-essigsäure. Man isoliert 1,03 g der Titelverbindung.

F: 180—190 (Z)
IR (KBr): 1770 $cm^{-1}$ (β-Lactam)
1725 $cm^{-1}$ (Acetat)
NMR (DMSO-$d_6$): δ=1,2 ppm (d, 6H, $CH(\underline{CH_3})_2$)
δ=2,0 ppm (s, 3H, $CO\underline{CH_3}$)
δ=6,75 ppm (s, 1H, Thiazol-H)

Beispiel 5

7-β-[α-syn-n-Butoximino-α-(2-amino-thiazol-4-yl)-acetamido]-cephalosporansäure

Gemäß Beispiel 1 B bei Verwendung von 1,22 g α-(2-Amino-thiazol-4-yl)-α-syn-n-butoximino-essigsäure. Man isoliert 0,82 g der Titelverbindung.

F: 250° C (Z)
IR (KBr): 1770 $cm^{-1}$ (β-Lactam)
1715 $cm^{-1}$ (Acetat)
NMR (DMSO-$d_6$): δ=0,7—1,8 ppm (m, 7H, $\underline{CH_2}-\underline{CH_2}-\underline{CH_3}$)
δ=2,05 ppm (s, 3H, $CO\underline{CH_3}$)
δ=6,75 ppm (s, 1H, Thiazol-H)

Beispiel 6

7-β-[α-syn-Methoximino-α-(2-amino-thiazol-4-yl)-acetamido]-3-(2-amino-1,3,4-thiadiazol-5-yl-thiomethyl)-3-cephem-4-carbonsäure

3,34 g des im Beispiel 1A Stufe 1 gewonnenen Gemisches werden zu einer Lösung aus 3,55 g 7-Amino-3-(2-amino-1,3,4-thiadiazol-5-yl-thiomethyl)-3-cephem-4-carbonsäure und 4,0 ml Triäthylamin in 40 ml trockenem Methylenchlorid gegeben. Nach Zugabe von 30 ml Tetrahydrofuran wird über Nacht bei Raumtemperatur gerührt.

Es wird eingedampft, in 60 ml $H_2O$ aufgenommen, vom Ungelösten abfiltriert, mit Essigester überschichtet und unter Eiskühlung mit 1 n-HCl auf pH 4,5 angesäuert. Man trennt die Phasen, extrahiert erneut mit Essigester bei 4,5 und säuert die wäßrige Phase im Eisbad auf pH 2,5 an.

Nach Absaugen und Trocknen über $P_2O_5$ gewinnt man 3,1 g der Titelverbindung.

F: 190—200 (Z)
IR (KBr): 1760 $cm^{-1}$ (β-Lactam)
NMR (DMSO-$d_6$): δ=3,8 ppm (s, 3H, $OCH_3$)
δ=5,7 ppm (q, 1H, 7-CH)
δ=6,73 ppm (s, 1H, Thiazol-H)
δ=7,4 ppm (breites Signal, 2H, $NH_2$)

Beispiel 7

7-β-[α-syn-Methoximino-α-(2-allylamino-thiazol-4-yl)-acetamido]-cephalosporansäure

1,69 g (7 mMol) α-syn-Methoximino-α-(2-allylaminothiazol-4-yl)-essigsäure werden in 50 ml wasserfreiem Tetrahydrofuran gelöst und mit 0,95 g (7 mMol) Hydroxybenztriazol versetzt. Man erwärmt auf 45° C, gibt 1,45 g (7 mMol) Dicyclohexylcarbodiimid hinzu und rührt 5 Stunden bei dieser Temperatur. Nach dem Erkalten wird der ausgefallene Dicyclohexylharnstoff abgesaugt. Die so erhaltene Lösung des aktivierten Esters tropft man bei Raumtemperatur zu einer Lösung von 1,91 g (7 mMol)

7-Aminocephalosporansäure und 3,36 ml Triäthylamin in 30 ml wasserfreiem Methylenchlorid. Man rührt über Nacht bei Raumtemperatur, saugt vom Ungelösten ab und dampft zur Trockne ein. Der Rückstand wird in Wasser gelöst und die Lösung bei pH 5 mit Essigester mehrfach gewaschen. Anschließend stellt man mit 1 n-Salzsäure auf pH 2,9 ein. Dabei fällt das Produkt aus, das abgesaugt, gut mit Wasser gewaschen und über Kaliumhydroxid getrocknet wird. Man erhält 1,78 g 7-β-[α-syn-Methoximino-α-(2-allylamino-thiazol-4-yl)-acetamido]-cephalosporansäure.

F: 165—170 (Z)
IR (KBr): 1775 $cm^{-1}$ (β-Lactam)
1730 $cm^{-1}$ ($OCOCH_3$)
NMR (DMSO-$d_6$): $\delta$=2,05 ppm (s, 3H, $OCO\underline{CH_3}$)
$\delta$=3,88 ppm (s, 3H, $OCH_3$)
$\delta$=6,8 ppm (s, 1H, Thiazol-H)

## Beispiel 8

### 7-β-[α-syn-Methoximino-α-(2-chloroacetamino-thiazol-4-yl)-acetamido]-cephalosporansäure

Gemäß Beispiel 7 bei Verwendung von 1,94 g α-(2-Chloracetylamino-thiazol-4-yl)-α-syn-methoximino-essigsäure. Man erhält 2,4 g der Titelverbindung.

F: 200 (Z)
IR (KBr): 1770 $cm^{-1}$ (β-Lactam)
1725 $cm^{-1}$ (Acetat)
NMR (DMSO-$d_6$): $\delta$=2,05 ppm (s, 3H, $CO\underline{CH_3}$)
$\delta$=3,9 ppm (s, 3H, $O\underline{CH_3}$)
$\delta$=4,4 ppm (s, 2H, $CO\underline{CH_2}Cl$)
$\delta$=7,45 ppm (s, 1H, Thiazol-H)

## Beispiel 9

### 7-β-[α-syn-Methoximino-(2-(5-amino-1,3,4-thiadiazol-2-yl-mercapto-acetamido)-thiazol-4-yl)-acetamido]-cephalosporansäure

Gemäß Beispiel 7 bei Verwendung von 2,6 g α-syn-Methoximino-[2-(5-amino-1,3,4-thiadiazol-2-yl-mercapto-acetamido)-thiazol-4-yl]-essigsäure. Man erhält 2,3 g der Titelverbindung.

F: 250 (Z)
IR (KBr): 1770 $cm^{-1}$ (β-Lactam)
NMR (DMSO-$d_6$): $\delta$=2,05 ppm (s, 3H, $COCH_3$)
$\delta$=3,95 ppm (s, 3H, $OCH_3$)
$\delta$=7,4 ppm (s, 1H, Thiazol-H)
$\delta$=7,2 ppm (breites Signal, 2H, $NH_2$)

## Beispiel 10

### 7-β-[α-syn-Methoximino-(2-(5-methyl-1,3,4-thiadiazol-2-yl-mercapto-acetamido)-thiazol-4-yl)-acetamido]-cephalosporansäure

Gemäß Beispiel 7 bei Verwendung von 2,6 g -syn-Methoximino-[2-(5-methyl-1,3,4-thiadiazol-2-yl-mercapto-acetamido)-thiazol-4-yl]-essigsäure. Man erhält 2,1 g der Titelverbindung.

F: 190—200 (Z)
IR (KBr): 1775 $cm^{-1}$ (β-Lactam)
NMR (DMSO-$d_6$): $\delta$=2,05 ppm (s, 3H, $COCH_3$)
$\delta$=2,7 ppm (s, 3H, $CH_3$)
$\delta$=3,95 ppm (s, 3H, $OCH_3$)
$\delta$=7,3 ppm (s, 1H, Thiazol-H)

## Beispiel 11

### 7-$\beta$-[$\alpha$-syn-Methoximino-$\alpha$-(2-amino-5-bromo-thiazol-4-yl)-acetamido]-cephalosporansäure

750 mg $\alpha$-syn-Methoximino-$\alpha$-(2-amino-5-bromo-thiazol-4-yl)-essigsäure, $^1/_2$ Mol Äthanol enthaltend, werden in 7 ml DMF gelöst, mit 30 ml Tetrachlorkohlenstoff versetzt und auf 7 ml eingedampft. Unter Rühren bei Raumtemperatur wird mit 0,373 g Hydroxybenzotriazol in 5 ml DMF und anschließend mit 0,5 g Dicyclohexylcarbodiimid versetzt.

Nach 2 Stunden Rühren bei Raumtemperatur wird das Gemisch mit einer Lösung aus 680 mg 7-Aminocephalosporansäure und 750 mg Triäthylamin in 25 ml Methylenchlorid versetzt und weitere 16 Stunden gerührt. Es wird vom Dicyclohexylharnstoff abfiltriert, das Methylenchlorid im Vakuum abgedampft und die verbleibende Lösung mit Wasser auf 70 ml verdünnt. Mit gesättigter $NaHCO_3$-Lösung wird auf pH 7,5 eingestellt, mit 20 ml Essigester 3 × extrahiert, nochmals filtriert und bei 0° C mit 2 n-HCl auf pH 2,0 gestellt. Nach einstündigem Rühren im Eisbad können 750 mg 7-[$\alpha$-syn-Methoximino-$\alpha$-(2-amino-5-bromo-thiazol-4-yl)-acetamido]-cephalosporansäure abfiltriert werden.

F: 140—150° (Z)
IR (KBr): 1770 $cm^{-1}$ ($\beta$-Lactam)
1720 $cm^{-1}$ (Acetat)
NMR: $\delta$ = 2,0 ppm (s, 3H, $OCO\underline{CH_3}$)
$\delta$ = 3,9 ppm [232 Hz] (s, 3H, $=N-O\underline{CH_3}$)
$\delta$ = 9,5 ppm (d, 1H, $-N\underline{H}-CO-$)

## Beispiel 12

### 7-$\beta$-[$\alpha$-syn-Methoximino-$\alpha$-(2-amino-5-chloro-thiazol-4-yl)-acetamido]-cephalosporansäure

1,42 g $\alpha$-(2-Amino-5-chloro-thiazol-4-yl)-$\alpha$-synmethoximino-essigsäure, 1 Mol Methanol enthaltend, wurden in 20 ml Tetrahydrofuran gelöst mit 30 ml Tetrachlorkohlenstoff versetzt und zur Trockne eingedampft. Der Rückstand wird in 20 ml Tetrahydrofuran gelöst und nach Zugabe von 670 mg Hydroxybenzotriazol auf 50° C erwärmt. Unter Rühren gibt man 1,03 g Dicyclohexylcarbodiimid, worauf nach ca. 1 Min. die Ausfällung von Dicyclohexylharnstoff beginnt.

Nach 4-stündigem Rühren saugt man den entstandenen Harnstoff ab, und tropft die Mutterlauge zu 1,36 g 7-Aminocephalosporansäure und 2,0 ml Triäthylamin, gelöst in 20 ml Methylenchlorid. Man beläßt über Nacht bei Raumtemperatur, dampft zur Trockne ein, nimmt in 30 ml $H_2O$ auf, überschichtet mit Essigester und säuert mit 2 n-HCl unter Eiskühlung auf pH 4,5 an. Man trennt die Phasen, extrahiert erneut mit Essigester und säuert die wäßrige Phase im Eisbad auf pH 2,2 an. Nach Absaugen und Trocknen über $P_2O_5$ gewinnt man 0,98 g 7-[$\alpha$-syn-Methoximino-$\alpha$-(2-amino-5-chloro-thiazol-4-yl)-acetamido]-cephalosporansäure.

F: 135—145 (Z)
IR (KBr): 1770 $cm^{-1}$ ($\beta$-Lactam)
1720 $cm^{-1}$ (Acetatbande)
NMR ($DMSO-d_6$): $\delta$ = 2,0 ppm (s, 3H, $OCO\underline{CH_3}$)
$\delta$ = 3,85 ppm [231 Hz] (s, 3H, $=NOCH_3$)
$\delta$ = 9,5 ppm (d, 1H, $N\underline{H}CO$)

## Beispiel 13

### 7-$\beta$-[$\alpha$-syn-Methoximino-$\alpha$-(2-amino-5-chloro-thiazol-4-yl)-acetamido]-cephalosporansäure

1,42 g $\alpha$-(2-Amino-5-chloro-thiazol-4-yl)-$\alpha$-synmethoximino-essigsäure, 1 Mol Methanol enthaltend, werden in 20 ml Tetrahydrofuran gelöst, und nach Zugabe von 670 mg Hydroxybenzotriazol unter Rühren mit 1,03 g Dicyclohexylcarbodiimid versetzt. Nach ca. 3 Min. beginnt die Ausfällung von Di-cyclohexylharnstoff. Die weitere Umsetzung und Aufarbeitung erfolgt wie in Beispiel 12 beschrieben. Man erhält so 1,2 g der Titelverbindung, in ihren physikalischen Konstanten, dünnschichtchromatographisch, NMR- und IR-spektroskopisch identisch mit dem nach Beispiel 12 gewonnenen Produkt.

## Beispiel 14

### 7-$\beta$-[$\alpha$-syn-Methoximino-$\alpha$-(2-amino-5-thiocyanato-thiazol-4-yl)-acetamido]-cephalosporansäure

645 mg $\alpha$-(2-Amino-5-thiocyanato-thiazol-4-yl)-$\alpha$-syn-methoximino-essigsäure werden in einer Mischung aus 2 ml Dimethylformamid und 15 ml Methylenchlorid aufgeschlämmt. Man versetzt mit 350 mg Hydroxybenzotriazol erwärmt zum Rückfluß und gibt nun 500 mg Dicyclohexylcarbodiimid hinzu. Man rührt bei Raumtemperatur 1 1/2 Stunden, trennt den Harnstoff durch Filtration ab und tropft eine Lösung aus 680 mg 7-Aminocephalosporansäure und 1,0 g Triäthylamin in 20 ml Methylenchlorid hinzu. Man rührt 5 Stunden bei Raumtemperatur, versetzt mit 20 ml $H_2O$, trennt die Phasen und extrahiert die organische Schicht erneut mit 10 ml $H_2O$. Die vereinigten wäßrigen Phasen werden auf pH 5 eingestellt, dreimal mit Essigester ausgeschüttelt und dann mit 2 n-HCl auf pH 2,3 angesäuert. Man extrahiert 3 × mit Essigester, vereinigt die organischen Phasen, unterschichtet sie mit 20 ml $H_2O$ und stellt mit gesättigter $NaHCO_3$-Lösung einen pH von 7 ein.

Die wäßrige Phase wird abgetrennt und gefriergetrocknet. Man erhält so 700 mg 7-[$\alpha$-syn-Methoximino-$\alpha$-(2-amino-5-thiocyano-thiazol-4-yl)-acetamido]-cephalosporansäure.

F: 210
IR (KBr): 1760 $cm^{-1}$ ($\beta$-Lactambande)
NMR (DMSO-$d_6$): $\delta = 2{,}0$ ppm (s, 3H, $OCO\underline{CH_3}$)
$\delta = 3{,}9$ ppm [236 Hz] (s, 3H, $=NO\underline{CH_3}$)
$\delta = 9{,}5$ ppm (d, 1H, $-N\underline{H}CO-$)

## Beispiel 15

### 7-$\beta$-[$\alpha$-syn-Methoximino-$\alpha$-(2-hydroxy-thiazol-4-yl)-acetamido]-cephalosporansäure

1,01 g (5 mMol) $\alpha$-syn-Methoximino-$\alpha$-(2-hydroxythiazol-4-yl)-essigsäure werden in 30 ml Tetrahydrofuran gelöst. Zu dieser Lösung gibt man 0,675 g (5 mMol) Hydroxybenztriazol, erwärmt auf ca. 40°C und versetzt mit 1,03 g (5 mMol) Dicyclohexylcarbodiimid. Man rührt 4 Stunden, saugt den entstandenen Harnstoff ab und tropft die klare gelbe Lösung zu 1,36 g (5 mMol) 7-Aminocephalosporansäure und 2,4 ml Triäthylamin gelöst in 25 ml Methylenchlorid. Man rührt 13 Stunden bei Raumtemperatur, filtriert von Ungelöstem ab und dampft ein. Der Rückstand wird in Wasser aufgenommen und bei pH 5 mit Essigester gewaschen. Mit 1 n-Salzsäure wird der pH-Wert 3 eingestellt und das Produkt mit Essigsäureester extrahiert. Nach Abdampfen des Lösungsmittels erhält man 1,22 g Rohprodukt, das noch Hydroxybenztriazol enthält. Zur Reinigung wird in 30 ml Aceton gelöst von Ungelöstem abfiltriert und auf die Hälfte eingeengt. Man beläßt einige Stunden bei Eisbadtemperatur und saugt dann das auskristallisierte Produkt ab. Man erhält 0,6 g 7-$\beta$-[$\alpha$-syn-Methoximino-$\alpha$-(2-hydroxy-thiazol-4-yl)-acetamido]-cephalosporansäure.

F: 158—160 (Z)
IR (KBr): 1775 $cm^{-1}$ ($\beta$-Lactam)
1725 $cm^{-1}$ ($OCOCH_3$)
NMR (DMSO-$d_6$): $\delta = 2{,}05$ ppm (s, 3H, $OCO\underline{CH_3}$)
$\delta = 3{,}9$ ppm (s, 3H, $OCH_3$)
$\delta = 6{,}53$ ppm (s, 1H, Thiazol-H)

## Beispiel 16

### 7-$\beta$-[$\alpha$-syn-Äthoximino-$\alpha$-(2-amino-thiazol-4-yl)-acetamido]-cephalosporansäure

#### Stufe 1

27,1 g $\alpha$-syn-Äthoximino-$\alpha$-(2-amino-thiazol-4-yl)-essigsäure wurden in 145 ml DMF gelöst und bei Raumtemperatur nacheinander mit 12,1 g 1-Hydroxy-benzotriazol und 18,8 g Dicyclohexylcarbodiimid versetzt.

Nach 2 Stunden wurde vom Harnstoff abfiltriert.

Das Filtrat wurde unter Eiskühlung und Rühren mit soviel $H_2O$ versetzt bis kein Niederschlag mehr entstand. Es wurde abgesaugt und getrocknet. Man erhielt so 19,8 g 1-[$\alpha$-syn-Äthoximino-$\alpha$-(2-amino-thiazol-4-yl)-acetoxy]-benzotriazol.

F: 118—120° (Z)

Stufe 2

2,72 g 7-Aminocephalosporansäure wurden in 40 ml Methylenchlorid mit 3,5 ml Triäthylamin zur Lösung gebracht und mit 3 g des in Stufe 1 gewonnenen Esters versetzt. Nach 10 Min. war eine klare Lösung entstanden. Es wurde 5¹/₂ Stunden nachgerührt. Nach Zutropfen von 25 ml 1 n-HCl wurde abgesaugt und der Niederschlag in Alkohol verrieben. Nach Absaugen und Trocknen konnten 1,9 g der Titelverbindung gesammelt werden, die dünnschichtchromatographisch IR- und NMR-spektroskopisch identisch mit dem in Beispiel 2 gewonnenen Produkt war.

## Beispiel 17

7-[α-syn-Methoximino-α-(2-amino-5-chloro-thiazol-4-yl)-acetamido]-desacetoxycephalosporansäure

268 mg α-syn-Methoximino-α-(2-amino-5-chloro-thiazol-4-yl)-essigsäure, 1 Mol Methanol enthaltend, wurden in 10 ml THF gelöst, und mit 135 mg 1-Hydroxy-benzotriazol sowie 216 mg Dicyclohexylcarbodiimid versetzt. Nach 1 h wurde vom Harnstoff abfiltriert. Die Mutterlauge wurde mit einer Lösung aus 214 mg 7-Amino-des-acetoxycephalosporansäure und 303 mg Triäthylamin in 10 ml Aceton/2 ml $H_2O$ versetzt und über Nacht bei Raumtemperatur gerührt. Die Reaktionslösung wurde mit 30 ml Essigester versetzt und unter Rühren auf pH 2,0 angesäuert. Der beim Einengen der Essigesterphase verbliebene Rückstand wurde in 2,5 ml 80% Ameisensäure gelöst, und unter kräftigem Rühren in eine Lösung aus 5,25 g Ammoniumsulfat in 11,25 ml $H_2O$ getropft. Es konnten so 120 mg der Titelverbindung als Formiat isoliert werden.

F: >200° (Z)
IR (KBr): 1760 $cm^{-1}$ (β-Lactam)
NMR (DMSO-$d_6$): δ = 1,98 ppm (s, 3H, $CH_3$)
δ = 3,83 ppm [230 Hz] (S, 3H, $O\underline{CH_3}$, syn)
δ = 9,43 ppm (d, 1H, $N\underline{H}CO$)

## Beispiel 18

7-[α-syn-Äthoximino-α-(2-amino-5-chloro-thiazol-4-yl)-acetamido]-cephalosporansäure

Gemäß Beispiel 12 bei Verwendung von 1,25 g α-syn-Äthoximino-α-(2-amino-5-chloro-thiazol-4-yl)-essigsäure. Man erhält 1,45 g der Titelverbindung.

10F: 140—150° (Z)
IR (KBr): 1775 $cm^{-1}$ (β-Lactam)
1725 $cm^{-1}$ (O-Acetat)
NMR (DMSO-$d_6$): δ = 1,25 ppm (t, 3H, $OCH_2\underline{CH_3}$)
δ = 2,0 ppm (s, 3H, $OCOCH_3$)
δ = 4,1 ppm [251 Hz] (q, 2H, $O\underline{CH_2}CH_3$)
δ = 9,7 ppm (d, 1H, $N\underline{H}CO$)

## Beispiel 19

7-β-[α-syn-Äthoximino-α-(2-amino-5-chloro-thiazol-4-yl)-acetamido]-desacetoxy-cephalosporansäure

1,87 g α-syn-Äthoximino-α-(2-amino-5-chloro-thiazol-4-yl)-essigsäure wurden zusammen mit 1,35 g 1-Hydroxybenzotriazol in 50 ml THF bei 50° gelöst und mit 2,06 g Dicyclohexylcarbodiimid versetzt. Nach 1 Stunde wird vom Niederschlag abfiltriert und mit einer Lösung aus 2,14 g 7-Amino-desacetoxy-cephalosporansäure und 3 g Triäthylamin in 50 ml THF/10 ml $H_2O$ versetzt. Es wurde über Nacht gerührt. Nach Verdünnen mit 60 ml Wasser wurden die organ. Lösungsmittel im Vakuum entfernt. Es wurde zweimal mit je 30 ml Essigester geschüttelt und die wäßrige Phase anschließend vorsichtig auf pH 4,5 angesäuert. Nach fünfmaliger Extraktion mit je 30 ml Essigester wurde weiter auf pH 2,5 angesäuert, abfiltriert und getrocknet.

Man erhielt so 1,1 g der Titelverbindung.

F: 130 (Z)
IR (KBr): 1775 $cm^{-1}$ (β-Lactam)

NMR ($DMSO-d_6$): δ = 1,3 ppm (tr, 3H, $CH_2\underline{CH_3}$)
δ = 2,0 ppm (s, 3H, $CH_3$)
δ = 3,3 ppm (breit, 2H, C−2−H)
δ = 4,10 ppm (q, 2H, N−O−$\underline{CH_2}CH_3$, syn)
δ = 5,10 ppm (d, 1H, C−6−H)
δ = 5,70 ppm (q, 1H, C−7−H)
δ = 7,35 ppm (breit, 2H, $NH_2$)
δ = 9,5 ppm (d, 1H, CONH)

## Beispiel 20

### 7-[α-syn-Äthoxycarbonyl-methoximino-α-(2-amino-5-chloro-thiazol-4-yl)-acetamido]-cephalosporansäure

2,75 g α-syn-Äthoxycarbonylmethoximino-α-(2-tritylamino-5-chloro-thiazol-4-yl)-essigsäure wurden zusammen mit 0,675 g 1-Hydroxybenzotriazol in 50 ml trockenem THF gelöst und mit 1,08 g Dicyclohexylcarbodiimid versetzt. Nach einstündigem Rühren bei Raumtemperatur wurden 0,83 g Dicyclohexylharnstoff abfiltriert. Die Mutterlauge wurde unter Rühren mit einer Lösung von 1,36 g 7-ACS und 1,52 g Triäthylamin in 30 ml Aceton/5 ml $H_2O$ versetzt. Nach Rühren über Nacht wurde eingeengt, in 50 ml Essigester aufgenommen und mit $H_2O$ bei pH 1,8 extrahiert. Nach Eindampfen der Essigesterphase verblieben 4,4 g eines braungefärbten Rückstandes. Dieser wurde in 12 ml einer 75% $HCO_2H$ 1 Stunde bei 50° gerührt, zweimal mit Toluol extrahiert und mit 1 g Aktiv-Kohle entfärbt. Die hellgefärbte Ameisensäurelösung wurde zu einer gerührten Lösung aus 26 g $(NH_4)_2SO_4$ in 56 ml Wasser getropft. Man erhielt so 0,8 g 7-[α-syn-Äthoxycarbonylmethoximino-α-(2-amino-5-chloro-thiazol-4-yl)-acetamido]-cephalosporansäure mit F = 175—180° (Z)

IR (KBr): 1765 $cm^{-1}$ (β-Lactam)
1725 $cm^{-1}$ ($OCOCH_3$ und $-CO_2C_2H_5$)
NMR ($DMSO-d_6$): δ = 9,5 ppm (d, 1H, CONH)
δ = 7,22 ppm (s, breit, $NH_2$)
δ = 5,7 ppm (q, 7—H)
δ = 5,1 ppm (d, 6—H)
δ = 4,82 ppm (A B, 2H, $CH_2O$)
δ = 4,65 ppm [279 Hz syn] (s, 2H, $CH_2CO_2$)
δ = 4,13 ppm (q, 2H, $OCH_2CH_3$)
δ = 3,5 ppm (d, 2H, 2—H)
δ = 2,0 ppm (s, 3H, $COCH_3$)
δ = 1,2 ppm (t, 3H, $OCH_2\underline{CH_3}$)

## Beispiel 21

### 7-[α-syn-Äthoxycarbonyl-methoximino-α-(2-amino-5-chloro-thiazol-4-yl)-acetamido]-desacetoxy-cephalosporansäure

Analog Beispiel 17, bei Verwendung von 310 mg α-syn-Äthoxycarbonylmethoximino-α-(2-amino-5-chloro-thiazol-4-yl)-essigsäure. Man isoliert so 210 mg der Titelverbindung als Formiat.

F: ca 150° (Z)
IR (KBr): 1750 $cm^{-1}$ (β-Lactam)
NMR ($DMSO-d_6$): δ = 1,19 ppm (t, 3H, $CH_2\underline{CH_3}$)
δ = 2,0 ppm (s, 3H, $\underline{CH_3}$)
δ = 4,67 ppm [280 Hz] (s, 2H, O−$\underline{CH_2}$−)
δ = 9,4 ppm (d, 1H, N$\underline{H}$CO)

## Beispiel 22

### 7-[α-syn-Methoximino-α-(2-amino-thiazol-4-yl)-acetamido]-3-[2-(2-thienyl)-1H-1,3,4-triazol-5-yl-thiomethyl]-3-cephem-4-carbonsäure

Gemäß Beispiel 6 bei Verwendung von 4,15 g 7-Amino-3-[2-(2-thienyl)-1H-1,3,4-triazol-5-yl-thiomethyl]-3-cephem-4-carbonsäure. Man isoliert 3,77 g der Titelverbindung.

IR (KBr): 1765 $cm^{-1}$ ($\beta$-Lactam)

NMR(DMSO-$d_6$): $\delta$ = 3,81 ppm (s, 3 H, N−O$\underline{CH_3}$)
$\delta$ = 5,06 ppm (d, 1 H, C−6−H)
$\delta$ = 5,70 ppm (q, 1 H, C−7−H)
$\delta$ = 6,68 ppm (s, 1 H, Thiazol-H)
$\delta$ = 7,14 ppm (t, 3 H, $NH_2$ + 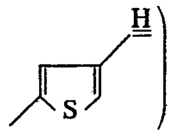)
$\delta$ = 7,62 ppm (d, 2 H, 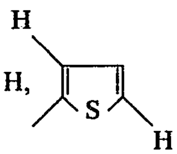)
$\delta$ = 9,5 ppm (d, 1 H, CON$\underline{\underline{H}}$)

## Beispiel 23

7-[$\alpha$-syn-Methoximino-$\alpha$-(2-amino-thiazol-4-yl)-acetamido]-3-(4,6-diamino-pyrimidin-2-yl-thiomethyl)-3-cephem-4-carbonsäure

Gemäß Beispiel 6 bei Verwendung von 3,7 g 7-Amino-3-(4,6-diamino-pyrimidin-2-yl-thiomethyl)-3-cephem-4-carbonsäure. Man isoliert 5,2 g der Titelverbindung.

IR (KBr): 1755 $cm^{-1}$ ($\beta$-Lactam)

NMR(DMSO-$d_6$): $\delta$ = 3,79 ppm (s, 3 H, $NOCH_3$)
$\delta$ = 5,07 ppm (m, 2 H, C−6−H + 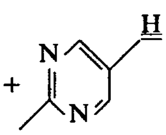)
$\delta$ = 5,65 ppm (q, 1 H, C−7−H)
$\delta$ = 6,8 ppm (s, 1 H, Thiazol-H)
$\delta$ = 9,5 ppm (d, 1 H, CON$\underline{\underline{H}}$)

## Beispiel 24

7-$\beta$-[$\alpha$-syn-Methoximino-$\alpha$-(2-amino-thiazol-4-yl)-acetamido]-3-(5-carboxymethyl-4-methyl-1,3-thiazol-2-yl-thiomethyl)-3-cephem-4-carbonsäure

Gemäß Beispiel 6 bei Verwendung von 4,2 g 7-$\beta$-Amino-3-(5-carboxymethyl-4-methyl-1,3-thiazol-2-yl-thiomethyl)-3-cephem-4-carbonsäure. Man isoliert 5,2 g der Titelverbindung.

IR (KBr): 1770 $cm^{-1}$ ($\beta$-Lactam)
15 NMR (DMSO-$d_6$): $\delta$=2,25 ppm (s, 3H, =C−$CH_3$)
$\delta$=3,75 ppm (s, 2H, −$\underline{CH_2}$−$CO_2H$)
$\delta$=3,85 ppm (s, 3H, NO$\underline{CH_3}$, syn)
$\delta$=6,75 ppm (s, 1H, Thiazol-H)
$\delta$=9,50 ppm (d, 1H, CON$\underline{H}$)

Beispiel 25

7-[α-syn-Methoximino-α-(2-amino-thiazol-4-yl)-acetamido]-3-(6-hydroxy-4-methyl-5-oxo-1,2,4-triazin-3-yl-thiomethyl)-3-cephem-4-carbonsäure

Gemäß Beispiel 6 bei Verwendung von 3,9 g 7-Amino-3-(6-hydroxy-4-methyl-5-oxo-1,2,4-triazin-3-yl-thiomethyl)-3-cephem-4-carbonsäure. Man isoliert 4,73 g der Titelverbindung.

IR (KBr): 1760 $cm^{-1}$ (β-Lactam)
NMR ($DMSO-d_6$): δ=3,28 ppm (s, 3H, N—$CH_3$)
δ=3,82 ppm (s, 3H, N—O—$CH_3$)
δ=5,75 ppm (q, 1H, C—7—H)
δ=6,72 ppm (s, 1H, Thiazol-H, syn)
δ=9,54 ppm (d, 1H, CONH)

Beispiel 26

7-[α-syn-Methoximino-α-(2-amino-thiazol-4-yl)-acetamido]-3-(1-methyl-1H-tetrazol-5-yl)-thiomethyl-3-cephem-4-carbonsäure

Gemäß Beispiel 6 bei Verwendung von 3,3 g 7-Amino-3-(1-methyl-1H-tetrazol-5-yl)-thiomethyl-3-cephem-4-carbonsäure. Man isoliert 4,32 g der Titelverbindung.

IR (KBr): 1760 $cm^{-1}$ (β-Lactam)
NMR ($DMSO-d_6$): δ=3,9 ppm (s, 3H, N—$CH_3$)
δ=3,82 ppm (s, 3H, N—O—$CH_3$)
δ=5,62 ppm (q, 1H, C—7—H)
δ=6,71 ppm (s, 1H, Thiazol-H)
δ=9,5 ppm (d, 1H, NHCO)

Beispiel 27

7-[α-syn-Methoximino-α-(2-amino-thiazol-4-yl)-acetamido]-3-(5-methyl-1,3,4-thiadiazol-2-yl)-thiomethyl-3-cephem-4-carbonsäure

Gemäß Beispiel 6 bei Verwendung von 3,5 g 7-Amino-3-(5-methyl-1,3,4-thiadiazol-2-yl)-thiomethyl-3-cephem-4-carbonsäure. Man isoliert 4,2 g der Titelverbindung.

IR (KBr): 1765 $cm^{-1}$ (β-Lactam)
NMR ($DMSO-d_6$): δ=2,68 ppm (s, 3H, $CH_3$)
δ=3,82 ppm (s, 3H, N—O—$CH_3$)
δ=5,78 ppm (q, 1H, C—7—H)
δ=6,74 ppm (s, 1H, Thiazol-H)
δ=9,5 ppm (d, 1H, CONH)

**Patentansprüche:**

1. Verfahren zur Acylierung von 7-Aminocephemderivaten, dadurch gekennzeichnet, daß man eine Acylierungskomponente der Formel I

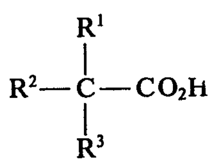

in der
$R^1$ Alkyl mit 1—4 C-Atomen, Phenyl, 2-Furyl oder Thiazolyl der Formel

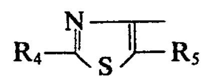

bedeutet, in der
$R_4$ für Hydroxy, Amino, Alkenylamino mit 2—4 C-Atomen, Formamino oder Acetamino, das durch Chlor, Brom, Fluor, Phenoxy oder durch Methyl oder Amino substituiertes 1,3,4-Thiadiazolylthio substituiert sein kann, steht und
$R_5$ Wasserstoff, Halogen, Cyano, Thiocyano, Hydroxy oder Alkoxycarbonyl mit 1—4 C-Atomen im Alkylteil sein kann,
$R_2$ und $R_3$ zusammen Sauerstoff oder eine Gruppe der Formel VIII

$$=N-X \qquad \text{(VIII)}$$

sein können, die vorzugsweise die syn-Konfiguration hat, und in der X für Hydroxy, Alkoxy mit 1—4 C-Atomen, Benzyloxy, p-Chlorbenzyloxy, Triphenylmethoxy, Aminocarbonylmethoxy, tert.-Butoxycarbonylmethoxy, Methoxycarbonylmethoxy, Äthoxycarbonylmethoxy, Cyanomethoxy, Allyloxy, Bromallyloxy, Phenoxy, oder Acyloxy mit 1—4 C-Atomen steht, mit einem 1-Hydroxybenzotriazol der allgemeinen Formel II

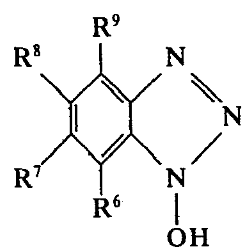

(II)

in der $R^6$, $R^7$, $R^8$ und $R^9$, die gleich oder verschieden sein können, Wasserstoff, Alkyl mit 1 bis 4 C-Atomen, Alkoxy mit 1 bis 4 C-Atomen, Halogen, Cyano, Nitro, Aminocarboxy, Alkylaminocarboxy mit 1 bis 4 Alkyl-C-Atomen, Aminosulfonyl, Alkylaminosulfonyl mit 1 bis 4 Alkyl-C-Atomen, Di-alkylaminosulfonyl mit 1 bis 4 Alkyl-C-Atomen, bedeuten, in ein aktives Derivat überführt und anschließend mit einem 7-Aminocephemderivat umsetzt.

2. Verfahren nach Anspruch 1, dadurch gekennzeichnet, daß als 7-Aminocephemderivat eine Verbindung der Formel IV

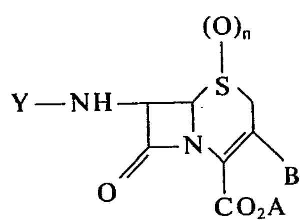

(IV)

eingesetzt wird, in der n für 0, 1 oder 2, A für Wasserstoff, ein Alkali- oder Erdalkalikation, ein Kation einer Ammoniumbase oder eine leicht entfernbare Estergruppe, Y für Wasserstoff oder eine Trialkylsilylgruppe steht und B Chlor, Brom, Methoxy oder eine Gruppe der Formel IX

$$-CH_2-B' \qquad \text{(IX)}$$

bedeutet, in der B' Wasserstoff, Hydroxy, Acyloxy mit 1—4 C-Atomen, Acylthio mit 1—4 C-Atomen, Aminocarbonyloxy, 1-Pyridinium oder —S-Het bedeuten, wobei Het für einen 5-gliedrigen heterocyclischen Ring stehen kann, der 1—4 Heteroatome aus der Gruppe Stickstoff, Schwefel und Sauerstoff enthalten und durch Amino, Alkyl mit 1—4 C-Atomen oder ein durch Carboxy oder Sulfonyl substituiertes Alkyl mit 1—4 C-Atomen oder durch Thienyl substituiert sein kann,
oder worin Het auch einen 6-gliedrigen heterocyclischen Ring bedeuten kann, der 1 bis 3 Stickstoffatome enthalten und durch Amino, Hydroxy, Oxo, Carboxy, Carboxymethyl, Alkyl mit 1—4 C-Atomen oder Alkoxy mit 1—4 C-Atomen substituiert sein kann, wobei ein solcher heterocyclischer Ring auch noch an einen weiteren heterocyclischen Ring ankondensiert sein kann.

3. Verfahren nach Anspruch 1, dadurch gekennzeichnet, daß man die Acylierungskomponente und ein gegebenenfalls substituiertes 1-Hydroxybenzotriazol mit Hilfe eines wasserentziehenden Mittels, insbesondere eines Carbodiimids, in ein aktiviertes Derivat überführt.

4. Verfahren nach Anspruch 1, dadurch gekennzeichnet, daß 1-Hydroxybenzotriazol eingesetzt wird.

**Claims:**

1. A process for the acylation of 7-amino-cephem derivatives, wich comprises converting an acylating component of the formula I

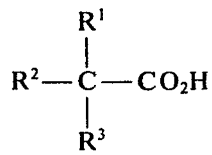

in which
$R^1$ is alkyl having from 1 to 4 carbon atoms, phenyl, 2-furyl or thiazolyl of the formula

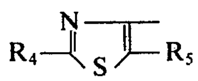

in which
$R_4$ is hydroxy, amino, alkenylamino having from 2 to 4 carbon atoms, formamino or acetamino optionally substituted by chlorine, bromine, fluorine, phenoxy or by 1,3,4-thiadiazolylthio in turn substituted by methyl or amino;
$R_5$ is hydrogen, halogen, cyano, thiocyano, hydroxy or alkoxycarbonyl having from 1 to 4 carbon atoms in the alkyl moiety;
$R^2$ und $R^3$ together are oxygen or a group of the formula VIII

$$=N-X \qquad \text{(VIII)}$$

which preferably has the syn configuration, and in which X is hydroxy, alkoxy having from 1 to 4 carbon atoms, benzyloxy, p-chlorobenzyloxy, triphenylmethoxy, aminocarbonylmethoxy, tert.-butoxycarbonylmethoxy, methoxycarbonylmethoxy, ethoxycarbonylmethoxy, cyanomethoxy, allyloxy, bromallyloxy, phenoxy or acyloxy having from 1 to 4 carbon atoms;
with a 1-hydroxybenzotriazole of the general formula II

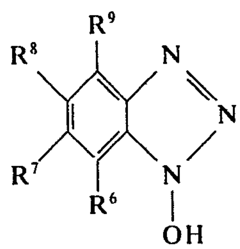

(II)

in which $R^6$ through $R^9$, being identical or different, each are hydrogen, alkyl having from 1 to 4 carbon atoms, alkoxy having from 1 to 4 carbon atoms, halogen, cyano, nitro, aminocarboxy, alkylaminocarboxy having from 1 to 4 carbon atoms in the alkyl moiety, aminosulfonyl, alkylaminosulfonyl having from 1 to 4 carbon atoms in the alkyl moiety, dialkylaminosulfonyl having from 1 to 4 carbon atoms in the alkyl moiety; to an active derivative, and subsequently reacting the latter one with a 7-aminocephem derivative.

2. A process as claimed in claim 1, wich comprises using as 7-amino-cephem derivative a compound of the formula IV

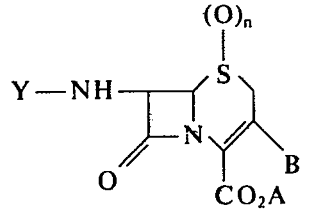

(IV)

in which n is 0, 1 or 2, A is hydrogen, an alkali metal or alkaline earth metal cation, a cation of an ammonium base or an easily removable ester group, Y is hydrogen or a trialkylsilyl group, and B is chlorine, bromine, methoxy or a group of the formula IX

$$—CH_2—B' \qquad (IX)$$

in which B′ is hydrogen, hydroxy, acyloxy having from 1 to 4 carbon atoms, acylthio having from 1 to 4 carbon atoms, aminocarbonyloxy, 1-pyridinium or —S-Het, where Het may be a 5-membered heterocycle optionally containing 1 to 4 hetero atoms selected from the group of nitrogen, sulfur and oxygen and optionally substituted by amino, alkyl having from 1 to 4 carbon atoms or alkyl having from 1 to 4 carbon atoms substituted by carboxy or sulfonyl, or substituted by thienyl, or may be a 6-membered heterocycle optionally containing from 1 to 3 nitrogen atoms and optionally substituted by amino, hydroxy, oxo, carboxy, carboxymethyl, alkyl having from 1 to 4 carbon atoms or alkoxy having from 1 to 4 carbon atoms; such heterocycle optionally being fused to a further heterocycle.

3. A process as claimed in claim 1, which comprises converting the acylating component and an optionally substituted 1-hydroxybenzotriazole to an active derivative with the aid of a dehydrating agent, especially of a carbodiimide.

4. A process as claimed in claim 1, which comprises using 1-hydroxybenzotriazole.

**Revendications**

1. Procédé d'acylation de dérivés de 7-aminocéphème, caractérisé en ce qu'on transforme un constituant d'acylation de formule I:

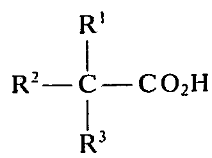

(I)

dans laquelle $R^1$ désigne un groupe alcoyle de 1 à 4 atomes de carbone, phényle, 2-furyle ou thiazolyle de formule:

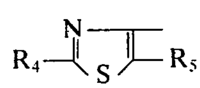

dans laquelle $R_4$ représente un groupe hydroxy, amino, alcénylamino de 2 à 4 atomes de carbone, formamino ou acétamino qui peut être substitué par du chlore, du brome, du fluor, un groupe phénoxy ou un groupe 1,3,4-thiadiazolylthio substitué par un groupe méthyle ou amino, et $R_5$ désigne l'hydrogène, un halogène ou un groupe cyano, thiocyano, hydroxy ou alcoxycarbonyle ayant de 1 à 4 atomes de carbone dans la partie alcoyle;
$R_2$ et $R_3$ peuvent être ensemble l'oxygène ou un groupe de formule VIII:

$$=N—X \qquad (VIII)$$

qui a de préférence la configuration syn, et dans laquelle X désigne un groupe hydroxy, alcoxy de 1 à 4 atomes de carbone, benzyloxy, p-chlorobenzyloxy, alcoxy de 1 à 4 atomes de carbone, benzyloxy, p-chlorobenzyloxy, triphénylméthoxy, aminocarbonylméthoxy, tert-butoxycarbonylméthoxy, méth-

oxycarbonylméthoxy éthoxycarbonylméthoxy, cyanométhoxy, allyloxy, bromallyloxy, phénoxy ou acyloxy de 1 à 4 atomes de carbone, avec un 1-hydroxybenzotriazole de formule II:

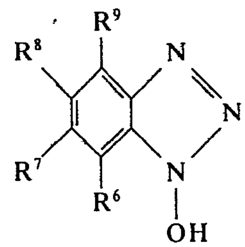

(II)

dans laquelle $R^6$, $R^7$, $R^8$ et $R^9$, qui peuvent être identiques ou différents, représentent l'hydrogène ou un groupe alcoyle de 1 à 4 atomes de carbone ou alcoxy de 1 à 4 atomes de carbone, un halogène, un groupe cyano, nitro, aminocarboxy, alcoylaminocarboxy ayant de 1 à 4 atomes de carbone dans le groupe alcoyle, aminosulfonyle, alcoylaminosulfonyle ayant de 1 à 4 atomes de carbone dans le groupe alcoyle, dialcoylaminosulfonyle ayant de 1 à 4 atomes de carbone dans le groupe alcoyle, en un dérivé activé, puis on le met à réagir avec un dérivé de 7-aminocéphème.

2. Procédé suivant la revendication 1, caractérisé en ce qu'on utilise comme dérivé de 7-aminocéphème un composé de formule IV:

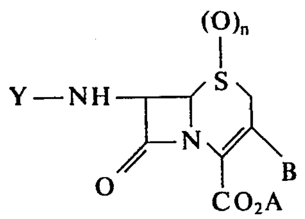

(IV)

dans laquelle n est égal à 0, 1 ou 2, A désigne l'hydrogène, un cation alcalin ou alcalino-terreux, le cation d'une base ammonium ou un groupe ester aisément éliminable, Y désigne l'hydrogène ou un groupe trialcoylsilyle et B désigne le chlore, le brome, un groupe méthoxy ou un groupe de formule IX:

$$—CH_2—B' \qquad (IX)$$

dans laquelle B' désigne l'hydrogène, un groupe hydroxy ou un groupe acyloxy de 1 à 4 atomes de carbone, acylthio de 1 à 4 atomes de carbone, aminocarbonyloxy, 1-pyridinium ou —S-Het, Het pouvant désigner un noyau hétérocyclique à 5 chaînons qui peut contenir de 1 à 4 hétéroatomes choisis parmi l'azote, le soufre et l'oxygéne et peut être substitué par un groupe amino, alcoyle de 1 à 4 atomes de carbone ou par un groupe alcoyle de 1 à 4 atomes de carbone substitué par des groupes carboxy ou sulfonyle, ou par un groupe thiényle, Het pouvant aussi désigner un noyau hétérocyclique à 6 chaînons pouvant contenir de 1 à 3 atomes d'azote et être substitué par des groupes amino, hydroxy, oxo, carboxy, carboxyméthyle, alcoyle de 1 à 4 atomes de carbone ou alcoxy de 1 à 4 atomes de carbone, le noyau hétérocyclique pouvant aussi être condensé à un autre noyau hétérocyclique.

3. Procédé suivant la revendication 1, caractérisé en ce qu'on transforme le constituant d'acylation et un 1-hydroxybenzotriazole éventuellement substitué, à l'aide d'un agent d'élimination de l'eau, en particulier d'un carbodiimide, en un dérivé activé.

4. Procédé suivant la revendication 1, caractérisé en ce qu'on utilise du 1-hydroxybenzotriazole.